# EUROPEAN PATENT APPLICATION

(11) **EP 0 750 037 A2**
(43) Date of publication of application: **27.12.1996**
(21) Application number: 96202206.7
(22) Date of filing: 08.06.1990
(51) Int. Cl.: C12N 9/64, C12Q 1/37

(54) **TNF-convertase and method measuring inhibition of TNF convertase**

(30) Priority: 16.08.1989 US 395253
(62) Divisional of application: 90917939.2
(71) Applicant: CETUS ONCOLOGY CORPORATION, Emeryville, CA 94608-2916 (US)
(72) Inventor: Kriegler, Michael, Rancho Santa Fe, California 92067 (US); Perez, Carl, Berkeley, California 94702 (US)
(74) Representative: Bizley, Richard Edward

(57) **Abstract**

The invention provides substantially purified TNF convertase, optionally said convertase being membrane associated and capable of converting TNF having a molecular weight of about 26,000 to one or more lower molecular weight TNF species, e.g. a lower molecular weight TNF species having a molecular weight of about 17,000 said convertase being inhibited by compounds selected from the group consisting of 3,4-dichloro-isocoumarin, elastinal and (1-((3-((acetyloxyl)-7-methoxy-8-oxy-8-oxo-5-thio-1-azabicyclo [4.2.0] oct-2-en-2-yl) carbonyl) morpholine, S,S-dioxide, (6R-cis).

## Description

This invention is in the area of immunology/ biochemistry, and particularly concerns the development of a TNF convertase which can be used in the identification of compounds that may be used to treat diseases, particularly sepsis, AIDS and autoimmune diseases, and thus afford the physician alternate treatment regimes.

In the United States alone nosocomial bacteremia develops in about 194,000 patients, and of these about 75,000 die. Maki, D.G., 1981, Nosocomial Infect., (Dikson, R.E., Ed.), page 183, Yrke Medical Books, U.S.A.. Most of these deaths are attributable to six major gram-negative bacilli, and these are Pseudomonas aeruginosa, Escherichia coli, Proteus, Klebsiella, Enterobacter and Serratia. The current treatment for bacteremia is the administration of antibiotics which, unfortunately, have limited effectiveness.

The precise pathology of bacteremia is not completely elucidated, nevertheless, it is known that bacterial endotoxins, lipopolysaccharides (LPS), are the primary causative agent. LPS consist of at least three significant antigenic regions, the lipid A, core polysaccharide, and O-specific polysaccharide. The latter is also referred to as O-specific chain or simply O-antigen. The O-specific chain region is a long-chain polysaccharide built up from repeating polysaccharide units. The number of polysaccharide units differs among different bacterial species and may vary from one to as many as six or seven monosaccharide units. While the O-specific chain varies among different gram-negative bacteria, the lipid A and core polysaccharides are similar if not identical.

Since LPS plays a key role in sepsis, a variety of approaches has been pursued to neutralize its activity. Presently, there is considerable work which suggest that antibody to LPS will soon be a valuable clinical adjunct to the standard antibiotic therapy.

LPS initiates a cascade of biochemical events that eventually causes the death of the patient. It is widely believed that the second event, after the introduction of LPS, is the production of tumor necrosis factor (TNF) as a result of LPS stimulation of macrophage cells. Thus, considerable effort has been expended to produce neutralizing antibody to TNF, or other molecules that could inhibit its septic effects. It is likely that antibody to TNF will have valuable clinical applications. Tracey, et al., 1987, Nature, 330:662.

TNF has been shown to exist in both membrane bound and soluble secreted forms. Decker, et al., 1987, J. of Immunol., 138:957; Kriegler, et al., 1988, Cell, 53:45. Human TNF has been cloned and shown to consist of a 17 kd polypeptide, plus an unusually long 76 amino acid putative signal leader sequence. The 17 kd molecule is a key agent involved in initiating the biochemical cascade responsible for sepsis. It has been proposed by Kriegler, et al., 1988, Cell, 53:45, that TNF may exist as both a membrane bound 26 kd form, and a soluble form corresponding to the 17 kd species. The 26 kd form is the precursor, or prohormone, of the mature 17 kd molecule. It has further been proposed by Kriegler, et al. above, that the two forms of TNF may have different biological effects.

It will be appreciated that because TNF plays a key role in causing sepsis that there is a need to identify and develop anti-TNF prophylactics/therapeutics. As mentioned above, anti-TNF antibody appears to be promising, and has been shown to be effective in baboons. However, these studies have involved the use of non-human TNF and non-human TNF antibody. From a practical standpoint non-human anti-TNF antibody will have limited therapeutic application because of immunologic rejection of the antibody by a patient's immune system. Consequently, human antibody, or genetically engineered antibody consisting of the human constant region and the mouse variable region are preferred.

TNF, in addition to playing a critical role in sepsis, has recently been shown to be involved in initiating the expression of human immunodeficiency virus in human cells that carry latent virus. Folks et al., Proc. Natl. Acad. Sci. USA, vol. 86, p. 2365 (1989). Thus, preventing or inhibiting the formation of the 17 kd, or lower molecular weight forms of TNF would be a valuable prophylactic for the treatment of AIDS patients by preventing the expression of virus that is latent in the patient.

TNF also plays a role in various autoimmune diseases, particularly arthritis. Duff, et al., 1987, International Conference on Tumor Necrosis Factor and Related Cytotoxins, vol. 175:10. Thus, compounds or methods for inhibiting TNF action will have considerable application for the treatment of a variety of diseases of immunologic origin.

In addition to antibody, other molecules with TNF inhibitory activity are being sought. Non-antibody TNF inhibitors are described by Seckinger, et al., 1988, J. Exp. Med., 167:151, and Seckinger, et al, 1989, J. Biol. Chem. 264:11966, and in European Patent Application No. 88830365.8, inventors Wallach, et al. The inhibitors are present in the urine of febrile patients, and have been purified and shown to have molecular weights of about 27,000-33,000. To date neither of the inhibitors have been shown to be effective in the treatment of sepsis.

From the foregoing discussion it is apparent that there is a need to identify and develop additional anti-TNF inhibitors, both antibody based or otherwise, that may be efficaciously applied in the treatment of sepsis.

In EP-A-90 917939.2, from which this case is divided, there is described, inter alia, a method that can be used to identify prophylactics and/or therapeutics for the treatment of sepsis premised on the ability of these medicaments to interfere with the cleavage of 26 kd TNF prohormone by an enzyme, termed convertase, that cleaves the 26 kd molecule thereby producing lower molecular weight sepsis inducing molecules, more particularly a method that identifies inhibitors of sepsis that have significant prophylactic and/or therapeutic applications premised on the ability of the inhibitors to interfere with the production of the 17 kd mature form of TNF from its 26 kd precursor by interfering with the activity of a convertase, that removes the 76 amino acid signal sequence from the 26 kd molecule to produce 17 kd TNF.

The present invention provides substantially purified TNF convertase, optionally said convertase being membrane associated and capable of converting TNF having a molecular weight of about 26,000 to one or more lower molecular weight TNF species, e.g. a lower molecular weight TNF species having a molecular weight of about 17,000 said convertase being inhibited by compounds selected from the group consisting of 3,4-dichloro-isocoumarin, elastinal and (1-((3-((acetyloxyl)-7-methoxy-8-oxy-8-oxo-5-thio-1-azabicyclo [4.2.0] oct-2-en-2-yl) carbonyl) morpholine, S,S-dioxide, (6R-cis).

In another aspect the invention provides a method of identifying prophylactics or therapeutics of an autoimmune disease, comprising the steps of:
(a) contacting TNF having a molecular weight of about 26,000 with an effective amount of TNF convertase, optionally said TNF and TNF convertase being in solution with a pH of about 7.0 and/or said TNF convertase being derived from HL60 cells;
(b) measuring the conversion of TNF having a molecular weight of about 26,000 to one or more lower molecular weight TNF species, optionally wherein said one or more lower molecular weight TNF species have molecular weights selected from about 17,000 and 15,000;
(c) repeating steps "a" and "b" above, and further including a molecule sought to be identified as a prophylactic or therapeutic of an autoimmune disease; and
(d) measuring the inhibition of said conversion of said TNF having a molecular weight of about 26,000 to one or more lower molecular weight TNF species.

Measuring inhibition of the convertase can comprise labelling the TNF with the molecular weight 26,000 with a suitable label e.g. a label selected from radionuclides, fluors, or enzymes. In some embodiments the autoimmune disease is arthritis.

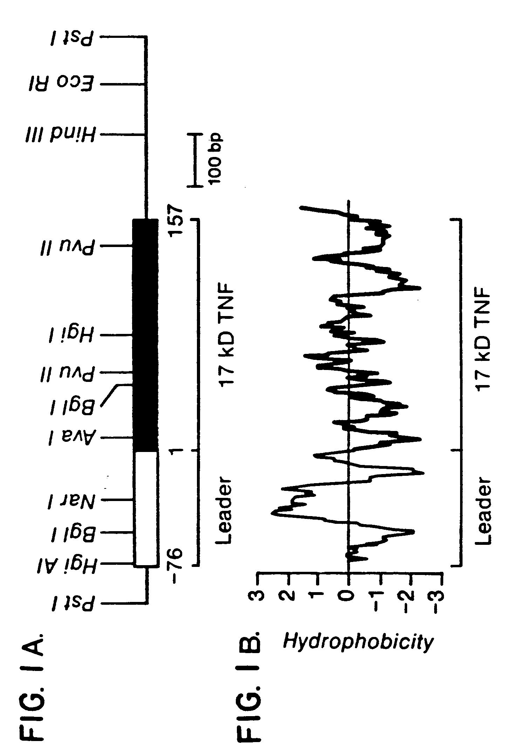

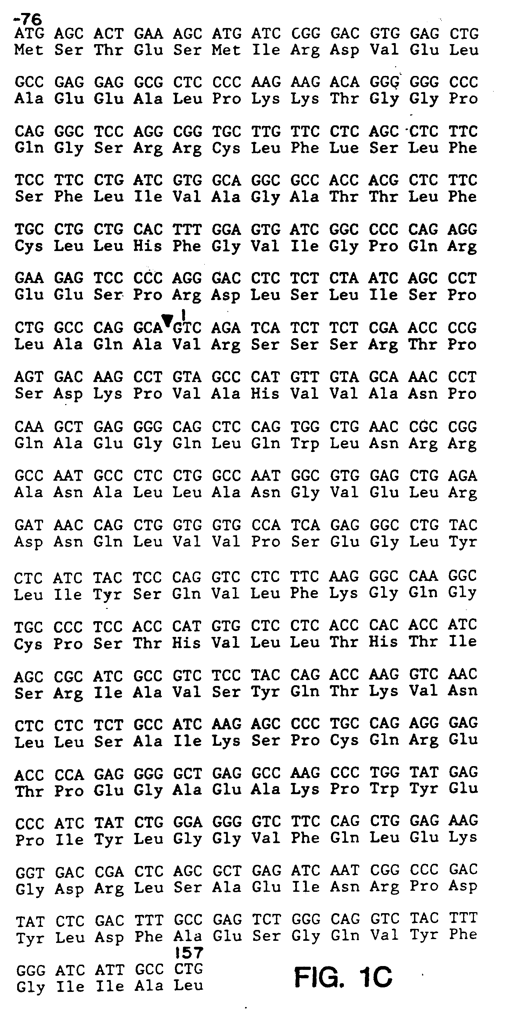

Figure 1, panel A, shows the restriction map of the DNA sequence that encodes 26 kd TNF. Panel B shows a hydrophobicity plot of 26 kd TNF, and panel C shows the DNA and amino acid sequences of the molecule.

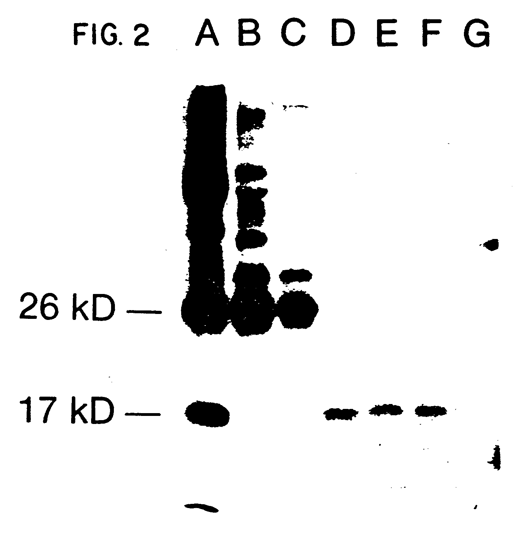

Figure 2 shows the conversion of 26 kd TNF by TNF convertase. Lanes A, B, and C show various controls: TNF 6.8 cell lysate (A), 26 kd transcription/translation (B) and incubation (C) controls. Lanes D, E, and F show the conversion of transcription/translation generated 26 kd TNF to predominately 17 kd TNF by convertase present in either HL 60 S-1 cytosol uninduced (D) and induced (E) fractions, or a P-1 pellet fraction prepared from induced cells. G is a blank lane.

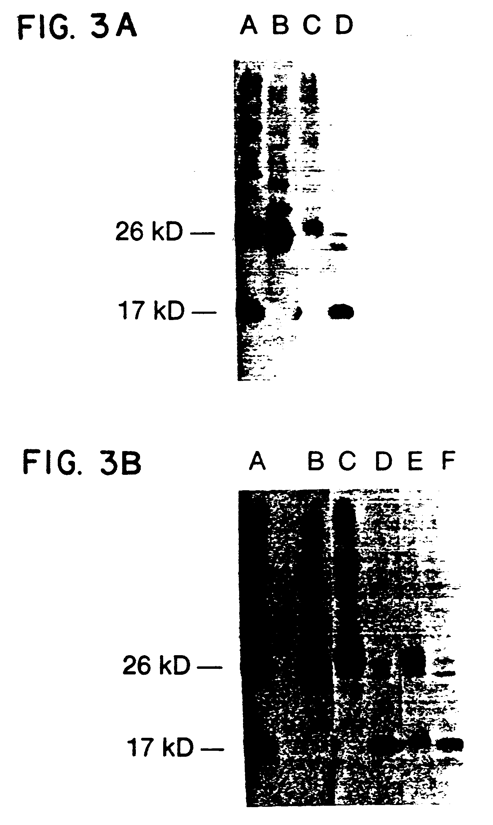

Figure 3 shows the effect of convertase inhibitors on the conversion of 26 kd TNF to its lower molecular weight forms as determined by gel electrophoresis. Lanes A,B,C,and D of panel 1 show, respectively; immunprecipitation of a cell lysate of the pFVXM-TNF6 transfected cell line TNF 6.8 (Kriegler, et al., 1988, in Cell, 53:45), immunprecipitation of in vitro transcribed/translated 26 kd TNF, the effect of (1-((3-((acetyloxyl)-7-methoxy-8-oxy-8-oxo-5-thio-1-azabicyclo [4.2.0] oct-2-en-2-yl) carbonyl) morpholine, S,S-dioxide, (6R-cis) on the conversion of 26 kd TNF, and the conversion of 26 kd TNF in the absence of (1-((3-((acetyloxyl)-7-methoxy-8-oxy-8-oxo-5-thio-1-azabicyclo [4.2.0] oct-2-en- 2-yl) carbonyl) morpholine, S,S-dioxide, (6R-cis). Lanes A and B of panel 2 show, respectively; immunprecipitation of a cell lysate of the pFVXM-TNF6 transfected cell line TNF 6.8 (Kriegler, et al., 1988, in Cell, 53:45), and immunprecipitation of in vitro transcribed/translated 26 kd TNF. Lanes C and D show the conversion of 26 kd TNF in the presence and absence of 3,4-dichloro-isocoumarin, respectively. Lanes E and F, show the conversion of 26 kd TNF in the presence and absence of elastinal, respectively.

To facilitate understanding the nature and scope of applicant's invention, several definitions regarding various aspects of the invention are presented below. It will be understood, however, that these definitions are general in nature, and encompassed within the definitions are meanings well known to those skilled in the art.

"Sepsis" is herein defined to mean a disease resulting from gram positive or gram negative bacterial infection, the latter primarily due to the bacterial endotoxin, lipopolysaccharide (LPS). It can be induced by at least the six major gram-negative bacilli and these are Pseudomonas aeruginosa, Escherichia coli, Proteus, Klebsiella, Enterobacter and Serratia. TNF is one factor that is detectable in the early phase of the disease, and that contributes to its progress. The 17 kd molecule, or shorter muteins known in the art, are the primary active species.

As used herein, TNF having a molecular weight of about 26,000, refers to the prohormone form of TNF. It is known that the amino-terminal peptide of the prohormone varies in length depending on the species from which it is derived, while the propeptide segment of the molecule is highly conserved. Indeed, in the mouse approximately 86% of the 79 amino acids that makeup the putative leader sequence of the pro-hormone are identical to the 76 known amino acids that comprise the pro-sequence of human TNF. Thus, it will be appreciated by those skilled in the art that when reference is made below to TNF having a molecular weight of about 26,000, that what is indicated is a molecule that is not derived from a particular species and that may have a slightly altered leader sequence compared to the human sequence as is known in the art.

The term "convertase", or "TNF convertase" is meant to encompass an enzyme normally present in the body that is responsible for cleaving 26 kd TNF to one or more lower molecular weight species. The convertase is substantially membrane associated, although significant activity is located in the cytosol. Another property of the convertase is that it is primarily associated with cells that produce TNF.

The phrase "membrane associated" as applied to TNF convertase indicates a form of the convertase that is substantially insoluble as indicated by the presence of most of the convertase activity in a 30,000 xg pellet fraction.

The assays described herein detect the enzymatic conversion of the 26 kd molecular weight form of TNF to, preferably, a 17,000 molecular weight form. The enzyme responsible for the conversion is terms a convertase. Thus, the present subject matter is most readily explained in four parts. Part one shows the materials and method for realizing the 26 kd form of TNF. Part two identifies sources of the TNF convertase of the invention, and methods for partially purifying the enzyme. Part three describes assays for detecting and identifying various convertase inhibitors. Finally, part four presents a description of ways of using the inhibitors to treat patients suffering from sepsis. Each of these sections will now be addressed separately.

Several patents/patent applications and scientific references are referred to below. The instant invention draws on some of the material and methods shown in these references, and thus it is intended that all of the references, in their entirety, be incorporated by reference.

It is important to note, and it should be stressed that virtually any procedure that enables one to measure the conversion of the 26 kd TNF species can be utilized to identify inhibitors of TNF. Thus, while the recombinant systems described below render the 26 kd molecule obtainable in considerable amounts, and therefore facilitate assaying for TNF inhibitors, it will be appreciated that non-recombinant systems may also be used. For instance, it has been shown that the 26 kd molecule can be identified in stimulated monocytes. This is described by Kriegler, et al., 1988, Cell, 53:45. Thus, a suitable assay procedure is to stimulate monocytes to produce the 26 kd molecule, and then in the presence of the convertase, measure the disappearance of the molecule to a lower molecular weight species, preferable the 17,000 molecular weight species.

Regarding the conversion of the 26,000 molecule, the convertase, as will become apparent below, cleaves the molecule at one internal site, and perhaps at several. The major site is at the junction which separates the secreted form of TNF, that is, the 17,000 molecular weight species, from the leader sequence. The sequence at this junction is -Gln-Ala-Val-Arg-Ser-Ser-. Thus, the major cleavage event occurs between alanine and valine, since valine is known to be the amino terminal amino acid of the 17,000 molecular weight molecule. Several other species of TNF are produced by the convertase, and thus these are the products of secondary cleavage sites. Regardless of whether the 26,000 molecule is cleaved at one or more sites, in so far as the identification of inhibitors of sepsis is concerned this is of marginal concern since the instant assay can monitor either the inhibition of the conversion of the 26,000 TNF species, or the appearance of a lower molecular weight form.

TNF, either the 26 kd or 17 kd forms, has been cloned and expressed in a number of systems. For instance, the cloning of rabbit TNF is disclosed in EP 146,026, published June 26, 1985 (Dainippon Pharmaceutical Co., Ltd.) and EP 148,311, published July 17, 1985 (Asahi Kasei Kogyo Kabushiki). The cloning of human TNF having 151 and 155 amino acids (2 and 6 less than the native form) is disclosed in EP 155,549, published September 25, 1985 (Dainippon Pharmaceutical Co., Ltd.), and human TNF having 155 amino acids is disclosed in EP 158,286, published October 16, 1985 (Asahi Kasei Kogyo Kabushiki Kaisha) and corresponding GB 1,158,829A, published November 20, 1985. The cloning of mature TNF (157 amino acids) and various modified forms (muteins) thereof is disclosed in EP 168,214, published January 15, 1986 (Genentech) and PCT US 85/01921, filed October 3, 1985, (Cetus Corporation). The latter, PCT, 85/01921, corresponds to WO 86/02381.

In addition, U.S. patents No. 4,677,063 and 4,677,064 show cDNA sequences that encode the 26,000 and 17,000 forms of TNF, as well as muteins of these molecules.

The cDNA sequence that encodes the 26 kd TNF species is preferably obtained from the plasmid, pB11, described in WO 86/02381; and U.S. Patents Nos. 4,677,063 and 4,677,064. The plasmid pB11 contains the SV40 promoter in operable linkage to the TNF coding sequence, and thus is useful for expressing the 26 kd TNF species in eukaryotic host cells. Additionally, a second plasmid which contains the entire sequence which encodes the 26 kd TNF species is described in the forgoing U.S. patent application and patents. It is designated pE4. The plasmid pE4 is on deposit with the American Type Culture Collection, Accession No. 39894.

The cDNA sequence that encodes the 26 kd TNF species is present in the plasmid pB11 as a Pst1 fragment. Thus, it is readily removed and inserted into any one of a number of suitable expression systems. The preferred expression system is the plasmid pFVXM, which is on deposit with the American Type Culture Collection and has Accession No. 67,103.

pFVXM is a retroviral vector that was derived from the plasmid pEVX described by Kriegler, et al., 1984, in Cell, 38:483. pEVX has a Moloney murine leukemia virus derived splice donor site 3' to the 5' - long terminal repeat. It was previously shown that this splice donor sequence decreases the yield of correctly spliced translational templates of retroviral constructions. Thus, pEVX was engineered to remove the splice donor site, and replaced with an analogous Sma I fragment of the Harvey murine sarcoma virus genome, which lacks the Moloney murine leukemia virus splice donor sequence. The resulting vector, pFVXM, lacks the Moloney murine leukemia virus spliced donor sequence and carries a vital packaging sequence. pFVXM has a convenient Pst I site in which the DNA sequences that encodes the 26 kd TNF species can be inserted.

A variety of biological materials are available as sources of convertase activity. These include tissues, cells, or extracts, or fluids associated therewith that are preferably, but not necessarily, of immunologic origin. Moreover, established cell lines may also be utilized. Suitable sources would include human peripheral blood mononuclear cells, such as leukocytes or cell lines of leukocyte origin, preferably the cell line HL60. Because of the ease of manipulating established cell fines, the preferred source of the convertase is HL60. Thus, the conversion of the 26 kd TNF species to the 17 kd species can be affected by combining the 26 kd species with either intact HL60 cells, extracts derived therefrom, or media in which the HL60 cells were grown and thus contains convertase activity. In some cell types, convertase activity is present in the culture medium after the appropriate stimulation, which is discussed more below. Further, because the convertase activity is partially membrane associated, it is possible to obtain a membrane fraction that may be utilized.

The procedures for isolating monocytes are well known in the art, as are other methods for culturing cell fines such as HL60. Briefly, monocytes may be prepared from peripheral blood by centrifugation first through Ficoll-paque and percoll (49.2%) using standard procedures. This yields an enriched population of monocytes and lymphocytes, and the monocytes can be further enriched by plating the mixture of cells onto tissue culture dishes and incubating the cells for a time sufficient to permit the monocytes to adhere to the surface of the dishes. The lymphocytes are then washed off of the plates leaving only adherent monocytes. These cells may then be used as is, or can be stimulated to produce enhanced levels of convertase using known monocytes activators, preferably lipopolysaccharide and phorbol myristate acetate. The cells may be fractionated, and either an extract or a membrane fraction prepared therefrom and employed in the assays described below.

Inhibitors of convertase activity will also be prophylactics or therapeutics that may be used in the treatment of sepsis. They may be identified using the foregoing assay, and further including in the assay reaction mixture compounds sought to be tested for inhibitory activity. A suitable assay would consist of combining 26 kd TNF, the convertase, and a putative inhibitor. It will be understood by those skilled in the art that the inhibitory material may be added to the convertase before the convertase is added to TNF, or it can be added to TNF prior to, or immediately after adding the convertase. The order of addition may facilitate identification of inhibitors, but it is not determinative. If a substance has inhibitory activity, this can be revealed by electrophoretic analysis of the solution which will reveal a reduction in the amount of the 26 kd species, and concomitantly the presence of lower molecular weight TNF molecules.

In addition to identifying compounds with unsuspected anti-convertase activity, several compounds will have strong inhibitory activity such as anti-convertase antibody, either polyclonal or monoclonal, or recombinant antibody, preferably humanized. Monoclonal antibody to the convertase may be produced using the general procedures described by Kohler, G. and Milstein, C., 1975, Nature, 256:495, which have been modified over the years as is known in the art. These initial studies involved fusing murine lymphocytes and drug selectable plasmacytomas to produce hybridomas. Subsequently, the technique has been applied to produce hybrid cell lines that secrete human monoclonal antibodies. The latter procedures are generally described in Abrams, P., 1986, Methods in Enzymology, 121:107, but other modifications are known to those skilled in the art. Regardless of whether murine or human antibody is produced, the antibody secreting cells are combined with the fusion partner and the cells fused with a suitable fusing agent, preferably polyethylene glycol, and more preferably polyethylene glycol 1000. The latter is added to a cell pellet containing the antibody secreting cells and the fusion partner in small amounts over a short period of time accompanied with gentle agitation. After the addition of the fusing agent, the cell mixture is washed to remove the fusing agent and any cellular debris, and the cell mixture consisting of fused and unfused cells seed into appropriate cell culture chambers containing selective growth media. After a period of several weeks, hybrid cells are apparent, and may be identified as to antibody production and subcloned to ensure the availability of a stable hybrid cell line.

The preferred antibody is human monoclonal antibody which can be prepared from lymphocytes sensitized with convertase either in vivo or in vitro by immortalization of antibody-producing hybrid cell lines, thereby making available a permanent source of the desired antibody. In vivo immunization techniques are well known in the art, while in vitro techniques are generally described by Luben, R. and Mohler, M., 1980, Molecular Immunology, 17:635, Reading, C. Methods in Enzymology, 121 (Part One): 18, or Voss, B., 1986, Methods in Enzymology, 121:27. A number of in vitro immunization systems have been shown to be effective for sensitizing human B-cells. Reading, C., 1982, J. of Immun. Methods, 53:261.

It will be apparent to those skilled in the art, that in lieu of immunizing individuals directly with convertase, lymphocytes may be isolated from individuals that are experiencing, or have experienced a bacteremic attack. A fraction of these lymphocytes will be sensitized to the convertase and can be used to produce permanent antibody secreting hybrid cell lines. For example, immunocompromised human patients are generally susceptible to bacterial infections, particularly those suffering from various malignancies, extensive burns, etc., and lymphocytes isolated therefrom may be a source of antibody secreting cells.

Sensitized lymphocytes can be immortalized by viral transformation. The preferred viral transformation technique for human lymphocytes involves the use of Epstein-barr virus. The virus is capable of transforming human B-cells, and has been used to generate human monoclonal antibodies. Crawford, D. et al., 1983, J. of General Virology, 64:697; Kozbor, V. and Roder, J., 1983, J. Immun. Today, 4:72.

Another procedure whereby sensitized lymphocytes may be immortalized consist of a combination of the above two techniques, that is viral transformation and cell fusion. The preferred combination consist of transforming antibody secreting cells with Epstein-barr virus, and subsequently fusing the transformed cells to a suitable fusion partner. The fusion partner may be a mouse myeloma cell line, a heteromyeloma line, or a human myeloma line, or other immortalized cell line. PCT Patent Application No. 81/00957; Schlom et al., 1980, PNAS USA, 77:6841; Croce et al., 1980, Nature, 288:488. The preferred fusion partner is a mouse-human hetero-hybrid, and more preferred is the cell line designated F3B6. This cell line is on deposit with the American Type Culture Collection, Accession No. HB8785. It was deposited April 18, 1985. The procedures for generating F3B6 are described in European Patent Application, Publication No. 174,204.

Techniques applicable to the use of Epstein-Barr virus transformation and the production of immortal antibody secreting cell lines are presented by Roder, J. et al., 1986, Methods in Enzymology, 121:140. Basically, the procedure consist of isolating Epstein-Barr virus from a suitable source, generally an infected cell line, and exposing the target antibody secreting cells to supernatants containing the virus. The cells are washed, and cultured in an appropriate cell culture medium. Subsequently, virally transformed cells present in the cell culture can be identified by the presence of the Epstein-Barr viral nuclear antigen, and transformed antibody secreting cells can be identified using standard methods known in the art.

It will be apparent to those skilled in the art that the antibody(s) may be altered and still maintain biological activity, e.g. antibody modified by reduction to various size fragments, such as F(ab')₂, Fab, FV, or the like. Also the hybrid cell lines that produce the antibody may be considered to be a source of the DNA that encodes the desired antibody, which may be isolated and transferred to cells by known genetic techniques to produce genetically engineered antibody.

An example of the latter would be the production of single chain antibody having the antibody combining site of the hybridomas described herein. Single chain antibody is described in U.S. Patent No. 4,704,692. A second example of genetically engineered antibody is recombinant, or chimeric antibody. Methods for producing recombinant antibody are shown in U.S. patent No. 4,816,567, inventor Cabilly, et al.; Japanese patent application, Serial No. 84169370, filed August 15, 1984; British patent application 8422238, filed on September 3, 1984; and Japanese patent application, No. 85239543, filed October 28, 1985. Also, British patent application, No. 867679, filed March 27, 1986 describes methods for producing an altered antibody in which at least parts of the complementary determining regions (CDRs) in the light or heavy chain variable domains have been replaced by analogous parts of CDRs from an antibody of different specificity. Using the procedures described therein it is feasible to construct recombinant antibody having the CDR region of one species grafted onto antibody from a second species that has its CDR region replaced. The preferred embodiment in this instance is a murine anti-convertase antibody CDR region that replaces the CDR region of human antibody.

In addition to antibody, compounds that compete with 26 kd TNF for binding to the convertase will inhibit or reduce the conversion of 26 kd TNF to the 17 kd form, and thus be useful medicaments for treating sepsis. One such class of reagents consists of peptides or proteins, or other compounds synthetic, or naturally occurring, that have similar convertase binding activity as the 26 kd form of TNF. Preferred within this class are peptides or proteins that have amino acid sequences similar to that found at the junction between 26 kd TNF and the 17 kd species. This sequence is Gln-Ala-Val-Arg-Ser-Ser-Ser, where Ala is the residue present on the portion of TNF remaining in the membrane after the cleavage event, and Val is the N-terminal amino acid of 17 kd TNF. It is important to note that while the sequence is shown to consist of seven amino acids that what is minimally intended is a dipeptide sequence that is recognized by the convertase, and which is Ala-Val. Thus while the dipeptide sequence is required as part of an inhibitory peptide or protein, the latter may also have additional amino acids that enhance, or are even required, for the dipeptide sequence to bind to the convertase and inhibit its enzymatic activity.

An alternate peptide/protein convertase inhibitor is one that has the amino acid sequence, a sequence that is functionally similar to: Gln-Ala-Val-Arg-Ser-Ser-Ser-Arg-Thr-Pro-Ser-Asp-Lys-Pro-Val-Ala. This peptide spans both convertase cleavage sites, and thus would prevent the formation of the 17 kd form of TNF and lower molecular weight forms. The first, and dominant cleavage site is between alanine and valine at positions -1 and +1; and the second site is between proline and valine at positions +12 and +13. These positions correspond to the amino acid sequence shown in Figure 1.

A second class of competitive inhibitors consists of compounds having the sequence shown above, that is, Gln-Ala-Val-Arg-Ser-Ser-Ser, or Gln-Ala-Val-Arg-Ser-Ser-Ser-Arg-Thr-Pro-Ser-Asp-Lys-Pro-Val-Ala but wherein amino acids have been mutated or deleted to yield a non-cleavable substrate. A preferred example of this peptide is a 26 kd non-cleavable TNF mutein produced by standard site specific mutagenesis techniques. Most preferred is a mutein wherein alanine and/or valine is substituted or deleted.

The peptides described above can be made by techniques well known in the art, such as, for example, the Merrifield solid-phase method described in Science, 232:341-347 (1985). The procedure may use commercially available synthesizers such as a Biosearch 9500 automated peptide machine, with cleavage of the blocked amino acids being achieved with hydrogen fluoride, and the peptides purified by preparative HPLC using a Waters Delta Prep 3000 instrument, on a 15-20 µm Vydac C4 PrepPAK column.

Finally, it is important to be cognizant of the fact that the specificity of the convertase is similar to enzymes like elastase, that is, enzymes that cleave preferably between neutrally charged amino acids such as between valine, proline, and alanine residues. Thus, in addition to the peptide inhibitors mentioned above, a variety of other inhibitors known to inhibit elastase will also generally inhibit the enzyme that cleaves TNF. Using the assay described below, those compounds that inhibit the convertase can be identified. A variety of elastase inhibitors are commercially available, see for example, Boehringer Mannheim Biochemicals catalogues, or are known in the art. Doherty, et al., 1986, Nature, 322:192; U.S. Patent Nos. 4,711,886; 4,797,396; 4,717,722; and 4,699,904. The preferred elastase inhibitors are modified cephalosporin antibiotics, such as those shown by Doherty, et al., above. More preferred is (1-((3-((acetyloxyl)-7-methoxy-8-oxy-8-oxo-5-thio-1-azabicyclo [4.2.0] oct-2-en- 2-yl) carbonyl) morpholine, S,S-dioxide, (6R-cis). Also, Stetler, et al., 1986, Nucleic Acids Research, 14:7883, describe a cDNA clone that codes for an inhibitor of neutrophil elastase.

Most of the recombinant techniques that are described herein that may be used to transform cells, fabricate vectors, extract messenger RNA, and the like are widely practiced in biotechnology and most practitioners are familiar with the standard materials and methods employed. However, for convenience, the following paragraphs are offered as a guideline.

Construction of suitable vectors containing the desired TNF coding sequence employs standard ligation and restriction techniques which are well understood in the art. Isolated vectors, DNA sequences, or synthesized oligonucleotides are cleaved, tailored, and religated in the form desired.

Site specific DNA cleavage is performed by treating with suitable restriction enzyme(s) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µ1 of buffer solution. In the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol followed by chromatography using a Sephadex G-50 spin column. If desired, size separation of the cleaved fragments may be performed by polyacrylamide gel or agarose gel electrophoresis using standard techniques. A general description of size separations is found in Methods in Enzymology, 1980, 65:499-560.

Restriction cleaved fragments may be blunt ended by treating with the large fragment of E. coli DNA polymerase I, that is, the Klenow fragment, in the presence of the four deoxynucleotide triphosphates (dNTPs) using incubation times of about 15 to 25 minutes at 20 to 25°C in 50 mM Tris pH 7.6, 50 mM NaCl, 6 mM MgCl₂, 6 mM DTT and 10 mM dNTPs. After treatment with Klenow, the mixture is extracted with phenol/chloroform and ethanol precipitated. Treatment under appropriate conditions with S1 nuclease results in hydrolysis of single-stranded portions.

Ligations are performed in 15-30 µl volumes under the following standard conditions and temperatures: 20mM Tris-Cl pH 7.5, 10 mM MgCl₂, 10 mM DTT, 33 µg/ml BSA, 10 mM-50 mM NaCl, and 1 mM ATP, 0.3-0.6 (Weiss) units T4 DNA ligase at 40°C for "sticky end" ligation, or for "blunt end" ligations. Intermolecular "sticky end" ligations are usually performed at 33-100 µg/ml total DNA concentration. In blunt end ligations, the total DNA concentration of the ends is about 1 µM.

In vector construction employing "vector fragments," the vector fragment is commonly treated with bacterial alkaline phosphatase (BAP) in order to remove the 5' phosphate and prevent religation of the vector. BAP digestions are conducted at pH 8 in approximately 150 mM Tris, in the presence of Na⁺ and Mg⁺² using about 1 unit of BAP per µg of vector at 60°C for about 1 hour. Nucleic acid fragments are recovered by extracting the preparation with phenol/chloroform, followed by ethanol precipitation. Alternatively, religation can be prevented in vectors which have been double digested by additional restriction enzyme digestion of the unwanted fragments.

In the constructions set forth below, correct ligations are confirmed by first transforming the appropriate E. coli strain with the ligation mixture. Successful transformants are selected by resistance to ampicillin, tetracycline or other antibiotics, or using other markers depending on the mode of plasmid construction, as is understood in the art. Miniprep DNA can be prepared from the transformants by the method of D. Ish-Howowicz et al., 1981, Nucleic Acids Res., 9:2989, and analyzed by restriction and/or sequenced by the dideoxy method of F. Sanger et al., 1977, Proc. Natl. Acad. Sci. (USA), 74:5463 as further described by Messing et al., 1981, Nucleic Acids Res., 9:309, or by the method of Maxam et al., 1980 Methods in Enzymology, 65:499.

Host strains used in cloning in M13 consists of E. coli strains susceptible to phage infection, such as E. coli K12 strain DG98 are employed. The DG98 strain has been deposited with ATCC July 13, 1984 and has accession number 1965.

Depending on the host cell used, transformation is done using standard techniques appropriate to such cells. Calcium treatment employing calcium chloride, as described by S. N. Cohen, 1972 Proc. Natl. Acad. Sci. (USA) 69:2110, or the RbCl₂ method described by Maniatis et al., 1984, Molecular Cloning: A Laboratory Manual, Cold Spring Harbor Press, p. 254, may be used for procaryotes. Transfection may also achieved using a modification of the calcium phosphate precipitation technique of Graham, F.L. et al., 1973 Virology 52:456 or Wigler, et al., 1978, Cell, 14:725.

Synthetic oligonucleotides were prepared by the triester method of Matteucci et al., 1981, J. Am Chem. Soc. 103:3185 or using commercially available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM MgCl₂, 5mM dithiothreitol, 1-2 mM ATP, 1.7 pmoles gamma ³²P-ATP (2.9 mCi/mmole), 0.1 mM spermidine, 0.1 mM EDTA.

Mutagenesis can be carried out using any number of procedures known in the art. These techniques are described by Smith, 1985, Annual Review of Genetics, 19:423, and modifications of some of the techniques are described in Methods in Enzymology, 154, part E, (eds.) Wu and Grossman (1987), chapters 17, 18, 19, and 20. The preferred procedure is a modification of the Gapped Duplex site-directed mutagenesis method. The general procedure is described by Kramer, et al., in chapter 17 of the Methods in Enzymology, above.

Conventional M13 mutagenesis methods involve annealing a short synthetic oligonucleotide to single stranded M13 DNA having a cloned target coding sequence that is sought to be mutagenized. The oligonucleotide is almost, but not entirely complementary to the target sequence and has at least one mispaired nucleotide. After the annealing reaction, the remaining portion of the single stranded DNA must be filled in to give heteroduplex DNA that can be transfected into a suitable host cell which allows for the expression of the mutation. In the gapped duplex method, a partial DNA duplex is constructed that has only the target region exposed, unlike the conventional methods which have the target region and the rest of the single stranded M13 DNA exposed. Like the conventional methods, a short oligonucleotide is annealed to the target region, and extended and ligated to produce a heteroduplex. However, because only a small portion of single-stranded DNA is available for hybridization in the gapped duplex method, the oligonucleotide does not anneal to undesired sites within the M13 genome. Further, this method has the additional advantage of introducing fewer errors during the formation of the heteroduplex since only a very small region of DNA on either side of the target region has to be filled in.

More specifically, the gapped duplex method involves cloning the target DNA sequence into an appropriate M13 phage that carries selectable markers, such as for example the stop codon amber mutation. The latter allows for negative selection in a host cell that cannot suppress the effects of the mutation. Preferably the phage is M13mp9 which contains two amber codons in critical phage genes. Thus, the sequence that encodes 26 kd TNF is cloned into M13mp9 amber+, and single stranded DNA is prepared therefrom using standard techniques. Next, double stranded replicative form DNA from M13 GAP, a genetically engineered M13 derivative that lacks the amber codons is cleaved with Hinc II restriction enzyme. The base sequence of M13 GAP is similar to M13mp18, which lacks both the amber codons and the sequence between base pairs 6172 and 6323. This deletion flanks the multiple cloning sites of the M13mp series and generates a unique Hinc II site. Gapped duplex DNA is formed, using standard DNA/DNA hybridization techniques, consisting of single stranded DNA hang the amber codons, and a second strand of DNA from Hinc II digested M13 GAP lacking both the amber codons and the TNF coding sequences. Thus, the only portion of the gapped duplex that is exposed is the 26 kd TNF target sequence. The desired oligonucleotide is annealed to the gapped duplex DNA , and any remaining gaps filled in with DNA polymerase and the nicks sealed with DNA ligase to produce a heteroduplex. The latter is transfected, preferably into a mismatch repair deficient host, and mixed phage produced. From the mixed phage population, phage carrying unmutated 26kd TNF DNA, which also have the amber mutations, can be selected against by infecting the mixed phage population into a host cell that cannot suppress the amber mutation. Clones can then be screened for phage that carry the desired TNF mutation.

Compounds identified as having convertase inhibitory activity will also have prophylactic or therapeutic applications in the treatment of sepsis. Because the onset of sepsis is associated with an increase in circulating TNF, these inhibitors may be used prophylactically in those instances where there is a risk of bacterial infection, particulary in a pre-operative setting. Similarly in those instances where there is an early diagnosis of sepsis, the inhibitors will have beneficial therapeutic effects in substantially reducing the amount of TNF that is produced.

A second medical application for inhibitors of convertase is for the treatment of AIDS. It has been shown that TNF causes the activation of latent human immunodeficiency virus. Folks et al., Proc. Natl. Acad. Sci. USA, vol. 86, p. 2365 (1989). Thus, preventing or inhibiting the formation of the 17 kd, or lower molecular weight forms of TNF by inhibition of the convertase would he a valuable prophylactic for the treatment of AIDS, and would preferable be used to treat patients that are infected with the virus that is in a latent phase.

Having generally described what the applicants believe their invention to be, presented below are examples that are illustrative of the scope of the invention. It will be appreciated by those skilled in the art that the examples are not intended to be construed as limiting the invention to the materials and methods shown as there are numerous substitutions that can be made therein without departing from the scope of the invention.

### Example 1

### Conversion of 26 kd TNF

The vector pFVXM, on deposit with the American Type Culture Collection, Accession No. 67,103, was used to produce a vector pFVXM-TNF6, which contains the DNA sequence that encodes the 26 kd TNF species. To produce the latter vector, the plasmid B11 which contains the cDNA sequence that encodes the 26 kd TNF species was treated with Pst I which excises the coding sequence. The fragment was purified using standard electrophoretic techniques. Next, the vector pFVXM was treated with Pst I, and the Pst I fragment from pB11 containing the 26 kd coding sequence was inserted into the polylinker region of the vector using standard techniques, as described above, to produce pFVX-TNF6. pFVX-TNF6 was used to produce the cell line TNF 6.8, as described by Kriegler et al, (1988), above, or as described in U.S. patent application titled "Cleavage Site Blocking Antibody to Prohormone Proteins and Uses Thereof," filed on the same day as the instant patent application. This application is Cetus Case No. 2534, inventors Kriegler and Perez.

TNF 6.8 expresses both 26 kd and 17 kd TNF. Figure 2 shows the conversion of 26 kd TNF by convertase activity present in HL 60 cells. The production of labelled 26 kd TNF by in vitro transcription/translation, and analysis by gel electrophoresis is described below, in Example 2. Note that the S-1 cytosol or pellet fractions cause the near complete conversion of 26 kd TNF to a 17 kd species. Figure 2 also shows, for comparative purposes, 26 kd and 17 kd TNF in a lysate of TNF 6.8 cells.

pFVXM and the plasmid pB11 were both amplified in E. coli strain HB101. Ligation of the fragments was carried out using standard conditions. Plasmid DNA was isolated after the ligation procedure and the correct orientation of the TNF encoding sequences was established by restriction analysis.

Plasmid DNA was prepared according to the procedure of Birnboim and Doly, as described in Nucleic Acid Research, 7:1513(1979). The plasmid DNA was banded twice in cesium chloride, and exhaustively dialyzed against TE buffer consisting of 10 mM Tris, pH 8.0, and 1 mM EDTA.

### Example 2

### Convertase Assay

### A. In Vitro Transcription/Translation Assay

The preferred assay procedure consists of in vitro transcription/translation of the 26 kd molecule, followed by treatment with convertase in the presence or absence of compounds being tested for convertase inhibitory activity. The procedure entails in vitro transcription/translation of the 26 kd molecule present in the plasmid B11. Thus, the sequence is removed from pB11 by Pst I digestion and inserted into the Pst I site of pGEM-3 (obtainable from Promega Biotec). The resulting plasmid, termed pGEM-TNF14, was amplified in E. coli using established techniques, and plasmid DNA prepared according to the procedure of Birnboim and Doly, described above. Plasmid DNA was in vitro transcribed by linearizing it with Hind III, and the linearized plasmid templates used to prepare capped transcripts with T7 RNA polymerase and an in vitro transcription kit supplied by Promega Biotec. Transcription was performed using standard techniques as suggested by the manufacturer's instructions.

The mRNA was in vitro translated in the presence of ³⁵S-cysteine to produce ³⁵S-cysteine labelled 26 kd TNF. The procedure consisted of using a rabbit reticulocyte lysate translation kit, also supplied by Promega Biotec, and following the conditions recommended by the manufacturer.

³⁵S-cysteine labelled 26 kd TNF was used to assay for convertase inhibitors as follows. 25 µl of in vitro translated material was combined with 250 µl of uninduced HL60 convertase activity, plus compounds sought to be assayed for inhibitory activity. The convertase was produced by harvesting 2 x 10⁹ HL60 cells, and isolating S-1 and P-30 fractions totalling 18 and 6 ml, respectively. 250 λ of the P-30 fraction was used, although the S-1 fraction may also be used. The assay was carried out at 30°C for 1 hour, essentially as described above. Next, the reaction mixture was immunoprecipitated with anti-TNF polyclonal antisera and protein A sepharose, pelleted and washed. The bound protein was eluted and electrophoresed. The gel was dried and exposed to x-ray film and subsequently developed. The gel electrophoretic profiles of 26 kd TNF treated with varying dilutions of HL60 convertase revealed those compounds with inhibitory activity.

Using the above assay, it was determined that 3,4-dichloro-isocoumarin and elastinal at concentrations of 100 µg/ml and 5 mg/ml, respectively, inhibit the convertase. It was also shown that (1-((3-((acetyloxyl)-7-methoxy-8-oxy-8-oxo-5-thio-1-azabicyclo [4.2.0] oct-2-en- 2-yl) carbonyl) morpholine, S,S-dioxide, (6R-cis) at a concentration of 1 mM inhibits convertase activity. These results are shown in figure 3.

### B. Monocyte Assay

In addition to 26 kd TNF produced by the in vitro transcription/translation assay described above, stimulated monocytes which produce 26 kd TNF, as described by Kriegler, et al., 1988, Cell, 53:45, and thus may be used as a source of the molecule. A suitable assay procedure is to stimulate monocytes, and then in the presence of the convertase, measure the disappearance of the 26 kd species to a lower molecular weight species, preferable 17 kd TNF.

Briefly, human monocytes are purified from human blood by centrifugation, and subsequently enriched for based on the adherence of monocytes to cell culture dishes. Centrifugation consist of purifying the monocytes through Ficoll-plaque and percoll (49.2%), obtainable from Pharmacia. The manufacturers recommended procedures are followed. Next, the mixture of cells resulting from the centrifugation step, consisting of monocytes and lymphocytes, are plated onto tissue culture dishes containing RPMI media supplemented with 20% fetal calf serum. The dishes are incubated for 30 minutes at 37°C after which they are extensively rinsed with the same media. This treatment removes non-adherent lymphocytes and leaves remaining only adherent monocytes.

Monocyte 26 kd TNF is radiolabelled as follows. The monocytes are incubated for 3 hours at 37°C in RPMI media supplemented with 20% fetal calf serum, 100 ng/ml lipolysaccharide, and 10 µg/ml phorbol myristate acetate for 30 minutes at 37°C. The latter two compounds induce the expression of TNF. The RPMI media is cysteine-minus, and the fetal calf serum present at a final concentration of 5%. The serum is dialyzed prior to use to remove any cysteine present. After the 30 minute incubation period, 100 uCi ³⁵S-cysteine is added and the cells are radiolabelled for 3 hours at 37°C after which they are lysed and used to assay for convertase activity. The steps for carrying out the assay, as well as identifying inhibitors of the convertase, are similar to those described above.

### Example 3

### TNF Mutein/Antibody/Peptide Inhibitors of Convertase Activity

The following compounds will have convertase inhibitory activity and can be prepared as follows, and tested for inhibitory activity as described above.

### A. Anti-Convertase Antibody

Antibody, either monoclonal or polyclonal, is prepared that either neutralizes the enzymatic activity of the convertase, or that binds to the convertase and thereby sterically prevents the convertase from binding to 26 kd TNF. The procedure consists of immunizing an appropriate host animal with a membranous fraction of HL60 cells producing the convertase. A sufficient amount of material should be used to elicit an immune response, and usually this will consist of between 10 µg to 10 mg per kilogram of body weight. Immunization may be conducted with adjuvant in a biologically acceptable buffer, as is known in the art. The best immunization route can be determined experimentally, and the primary immunization may be followed by one or more secondary immunizations depending on the strength of the immune response to the initial immunization. The presence of neutralizing anti-convertase antibody in the sera may be detected using the convertase assay described above wherein antisera is present in the assay mixture. Inhibition of the conversion of the 26 kd TNF species to a lower molecular weight species indicates the presence of neutralizing antibody. It is, of course, assumed that the proper controls are conducted to insure that anti-sera from non-immunized animals is not inhibitory. Polyclonal antibody may be purified as described below.

Monoclonal antibody to the convertase may be produced using either in vivo or in vitro immunization techniques, and sensitized lymphocytes resulting therefrom can be used to prepare hybrid cell lines that secrete the appropriate monoclonal antibody. Rodent, preferably of murine origin, or human antibody is most preferred. The in vivo immunization procedure involves sensitizing lymphocytes to the convertase by immunizing either mice or humans, and isolating therefrom the antibody secreting cell fraction and immortalizing the cells therein by one of several procedures. An alternate embodiment is to isolate lymphocytes that have already been sensitized to the convertase from septic patients, as described above.

### (i) Murine Antibody

For in vivo immunization of mice, the procedure of Kohler and Milstein described in Nature, 256:495 (1975) may be followed, or modified procedures such as those shown by Fendly, et al., 1987, Hybridoma, 6:359; Buck, et al., 1988, In Vitro, 18:377. In vitro techniques are generally described by Luben, R. and Mohler, M., 1980, Molecular Immunology, 17:635, Reading, C. Methods in Enzymology, 121 (Part One):18, or Voss, B., 1986, Methods in Enzymology, 121:27.

Mice are immunized with 1 mg/ml of a membranous fraction of HL-60 cells previously shown to be positive for convertase activity. The immunization is carried out in complete Freund's adjuvant. Two additional immunizations, or boosts, are performed at monthly intervals without adjuvant, and one month after the last boost the mice are given an I.V. boost of 10 ug of membranous material. The days after the I.V. boost, mice are sacrificed, their spleens removed, and the spleenocytes isolated and fused to an immortalized drug selectable myeloma partner cell fine. Numerous such myeloma lines are known in the art, most of which are incapable of growth in HAT supplemented cell culture media. A typical myeloma cell line is SP-2/0Ag 14. Thus, the hybridomas are formed by combining splenocytes and myeloma cells in a 5:1 ratio, which generally consists of 2 x 10⁶ myeloma cells to 1 x 10⁷ splenocytes. The cell mixture is pelleted, media removed and fusion affected by the addition of 1.0 ml of 40% (v/v) solution of polyethylene glycol 1500 by dropwise addition over 60 seconds at room temperature, followed by a 60 second incubation at 37°C. To the cell suspension with gentle agitation is added 9 ml of Dulbecco's Modified Eagles medium over 5 minutes. Cell clumps in the mixture are gently resuspended, the cells washed to remove any residual PEG and plated in microtiter plates at about 2 x 10⁵ cells/well in DMEM supplemented with 20% fetal calf serum. After 24 hours, the cells are fed a 2 x solution of hypoxanthine and azaserine selection medium.

Media from wells that exhibit positive cell growth may be screened for neutralizing monoclonal antibody to the convertase. The preferred assay is the convertase assay described in Example 2, above, wherein media sought to be tested for antibody activity is present in the assay. More preferred is to combine culture supernatants from 3-8 microtiter wells, and assay the mixture. If the mixture is positive, then media from each well may be assayed independently to identify the secreting hybridoma(s) Many assays are known in the art and can detect soluble, or non-soluble antigens, and are shown by Langone, J. and Van Vinakis, H., Methods of Enzymology, 92, Part E (1983).

Regardless of whether the antibody is polyclonal or monoclonal it is desirable to purify the antibody by standard techniques as is known in the art, or described by Springer, 1980, Monoclonal Antibodies,:194, (Eds. Kennett, T. McKearn and K. Bechtol, Plenum Press, New York. Generally this consists of at least one ammonium sulfate precipitation of the antibody using a 50% ammonium sulfate solution. Antibody affinity columns may also be used.

### (ii) Human Monoclonal Antibody

Peripheral blood lymphocytes are isolated from septic patients, and then infected with Epstein-Barr virus and the infected lymphocytes immortalized by fusion to a selectable myeloma cell line, and the hybrid cell lines so generated isolated and characterized as to antibody production.

More specifically, mononuclear cells are separated on Ficoll-hypaque (Pharmacia), and monocytes depleted from the mixture by adherence to plastic. Standard laboratory techniques were utilized to effect these procedures. Next, nonadherent cells are enriched for antibody producers by antigen-specific panning. Panning is a technique generally known in the art, and involves incubation of a population of antibody secreting cells on a plastic surface coated with the appropriate antigen. Those cells that express antibody on their surface bind antigen, and consequently adhere to the plastic surface, whereas cells that do not express cell surface antibody, do not adhere and can be removed by washing. Thus, specific antibody secreting cells are enriched for by this technique.

More specifically, 6-well plates (Costar) are coated with a membrane fraction containing convertase prepared from either induced or uninduced HL60 cells, as described above, such that 150 µg of membranous material is coated per well in phosphate buffered saline at 4°C overnight. The wells are blocked after the overnight incubation period with phosphate buffered saline containing 1% bovine serum albumin for at least 1 hour at 4°C, and subsequently washed with phosphate buffered saline/BSA. Next, 10⁷ lymphocytes in 1 ml of PBS/BSA are added to each well of the six well plates. The lymphocytes are allowed to incubate on the plates for 70 minutes, after which any nonadherent cells are removed by aspiration. The adherent cells are incubated with cell culture medium (IMDM, Sigma Chemical Co., St Louis, Missouri) containing 10% fetal calf serum.

The adherent cells are subjected to Epstein-Barr virus transformation by adding an equal amount of culture media obtained from growing the Epstein-Barr virus infected marmoset cell line, B95-8, and thus containing the virus, to media bathing the adherent cells. The cells were cultured in this environment at 37°C for 3 hours, and in this way the lymphocytes in the adherent cell population are subjected to Epstein-Barr infection. Following the infection period, the cells are washed and plated onto 96 well microtitre plates at a density of about 10⁴ - 10⁵ cells/well in IMDM medium, plus 10% fetal calf serum, and 30% conditioned medium. The latter is derived from a lymphoblastoid cell line, preferably JW5. The medium also contains 5 x 10⁻⁵ M 2-mercaptoethanol, 50 µg/ml gentamycin sulfate (Sigma), and 600 ng/ml cyclosporine A (Sandimmun, Sandoz, Basel, Switzerland).

After about 14 to 21 days of incubation, cell culture supernatants are combined and screened for convertase neutralizing activity as described above. Positive hybridomas are subcultured at low density, retested for neutralizing antibody, and grown up and fused to the cell line F3B6 using polyethylene glycol and the plate fusion technique known in the art. The latter technique is described by Larrick, J.W., (1985) in Human Hybridomas and Monoclonal Antibodies, E.G. Engleman, S.K.H. Foung, J.W., Larrick, and A.A. Raubitschek, Editors, Plenum Press, New York, page 446. F3B6 is a heteromyeloma cell line that is sensitive to growth in media containing 100 µM hypoxanthine, 5 µg/ml azaserine and 5 µM ouabain. Finally, the resulting hybrids are again screened to insure that they produce neutralizing anti-convertase antibody.

### B. 26 kd Non-Cleavable Muteins

26 kd TNF muteins are described that compete for binding to the convertase, thereby inhibiting or reducing its activity. The preferred embodiment muteins are those having valine at positions 1 and/or 13; or alanine at position -1 and/or proline at position 12, replaced/ or deleted. The muteins are constructed using a modification of the site directed mutagenesis gapped duplex method.

The following solutions/buffers are used to perform the desired procedures:
5 x Gapped duplex buffer (GDB) consisting of 0.938 M KCl, 0.063 M Tris, pH 7.5; 10 x PEL consisting of 1.0 M KCL, 0.30 M Tris, 0.15 M MgCl₂, 0.02 M DTT, pH 7.5; 10 x KB consisting of 0.50 M Tris, 0.10 M MgCl₂, 0.05 M DTT, 0.001 M EDTA, pH 8.0; a solution containing 0.25 mM dCTP, dATP, dGTP, dTTP, made fresh from 10 mM stocks; an ATP solution consisting of 0.1 M ATP made by dissolving 60 mg of ATP in 0.80 ml of H₂O and adjusting the pH to 7.0 with 0.1 M NaOH in a final volume of 1.0 ml with H₂O; 20% PEG/2.5 M NaCl; 3.0 M NaOAc; and TE Saturated Phenol.

Various bacterial strains and phage are employed to yield the desired muteins and these are BMH 71-18, JM103 for growing phage strains; HB2154: MutL, Su-, made competent for DNA transformation; and HB2151: Su-, used as lawn cells during transformation; M13 GAP, the RF is used for the formation of the gapped duplex; and M13mp19amber, the 26 kd TNF target DNA is cloned in this vector, and ssDNA isolated for the formation of gapped duplex.

Phage are infected into an appropriate bacterial strain, grown up, and titered as follows. In making a large-scale preparation of either phage for ssDNA or cells for dsDNA, or RF DNA, the same infection protocol is used.

Plaque purified phage is produced using standard techniques. Briefly, this consists of streaking phage supernatants on agar plates, followed by careful overlay with 4.0 ml of soft agar and 100 µl of fresh overnight culture of BMH 71-18. Next, isolated plaques are picked and incubated with a 1:50 dilution of fresh overnight culture of BMH 71-18 in R26 or R17 + 10 mM MgCL₂ with shaking at 37°C for 4.5-6 hours. R17 (N-Z amine broth) consist of 10 g N-Z amine, type A, 5 g NaCl with H₂O to 1 liter, while R26 consist of 8 g tryptone, 5 g yeast extract, 5 g NaCl, with water to 1 liter (YT broth). The phage stock is titered, and phage infected into bacteria at a multiplicity of infection (MOI) of 10. After incubating the culture with shaking at 37°C for 5 hours the cell suspension is pelleted, and the supernatant saved for ssDNA isolation, and the cells for RF isolation. RF DNA is isolated using established plasmid DNA isolation techniques, while ssDNA is isolated as follows.

250 ml of phage supernatant is spun down hard, after which 200 ml of the supernatant is decanted, followed by adding 50 ml of 20% PEG/2.5 M NaCl, and incubation overnight at 4°C, or on ice for 30 minutes. This mixture is also spun down hard, and the supernatant decanted. The bottle is spun again to pellet the phage precipitate along the sides of the bottle, and the remaining fluid aspirated with a Pasteur pipette. The pellet is resuspended in 5.0 ml of 1 x TE, and stored at 4°C, after which 0.5 ml of is extracted twice with 0.5 ml of TE saturated phenol. To the aqueous layer is added 0.050 ml of 3.0 M NaOAc and 1.0 ml 95% ethanol. The mixture is placed in a dry ice bath for 10 minutes, and centrifuged for 10 minutes in a microfuge at 4°C. The pellet is dried, and resuspended in 200 µl of 1X TE. This material may be stored in 0.050 ml aliquots at -20°C until used in the mutagenesis of 26 kd TNF.

The following oligonucleotides are used to change valine at positions 2 and/or 13, alanine at position -1 and proline at position 12. The oligonucleotides and their corresponding muteins are shown in Table 1.

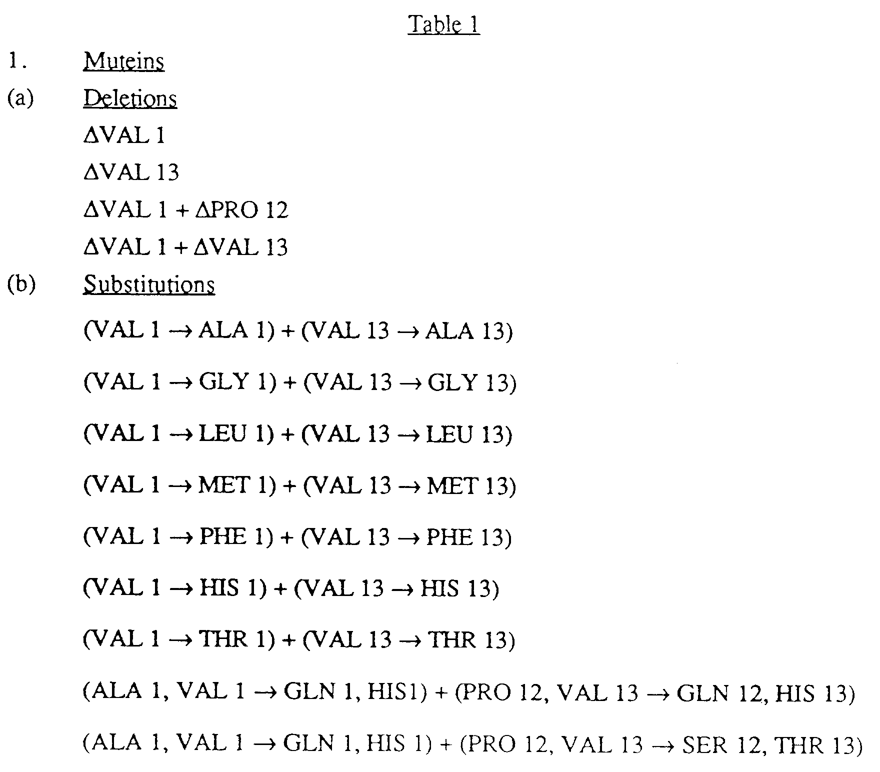

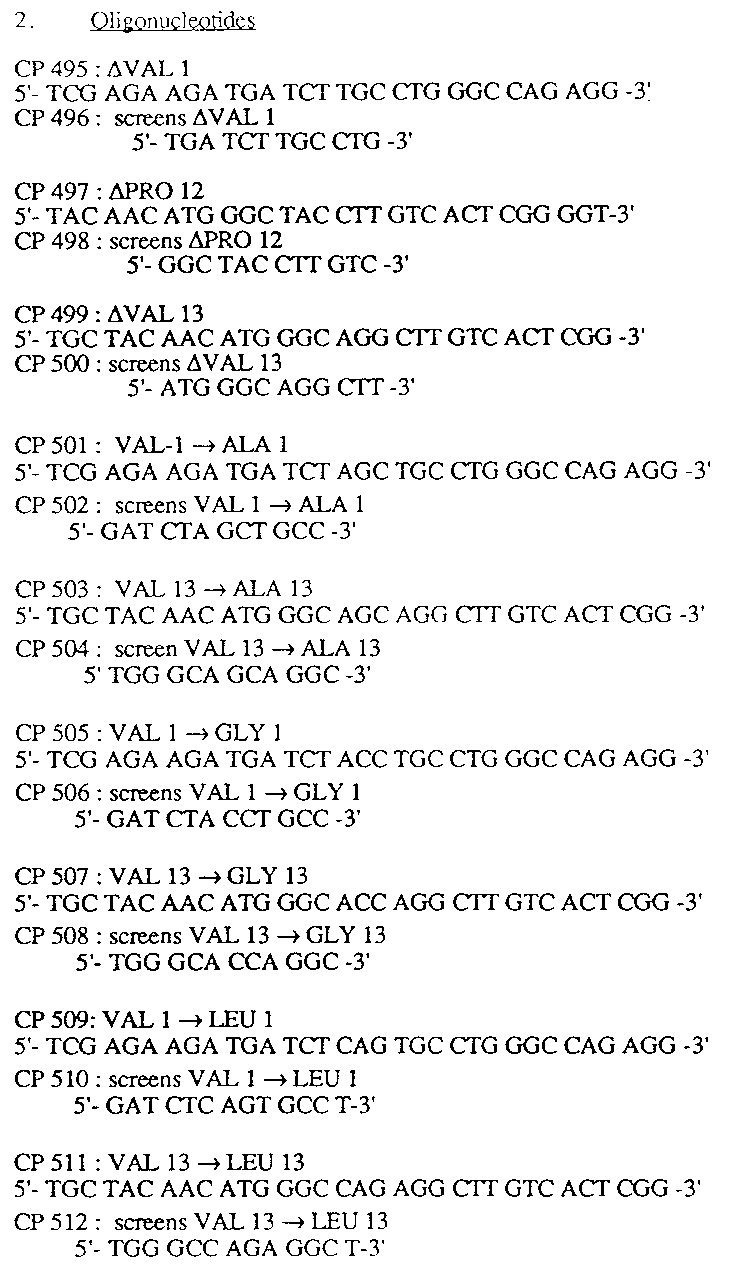

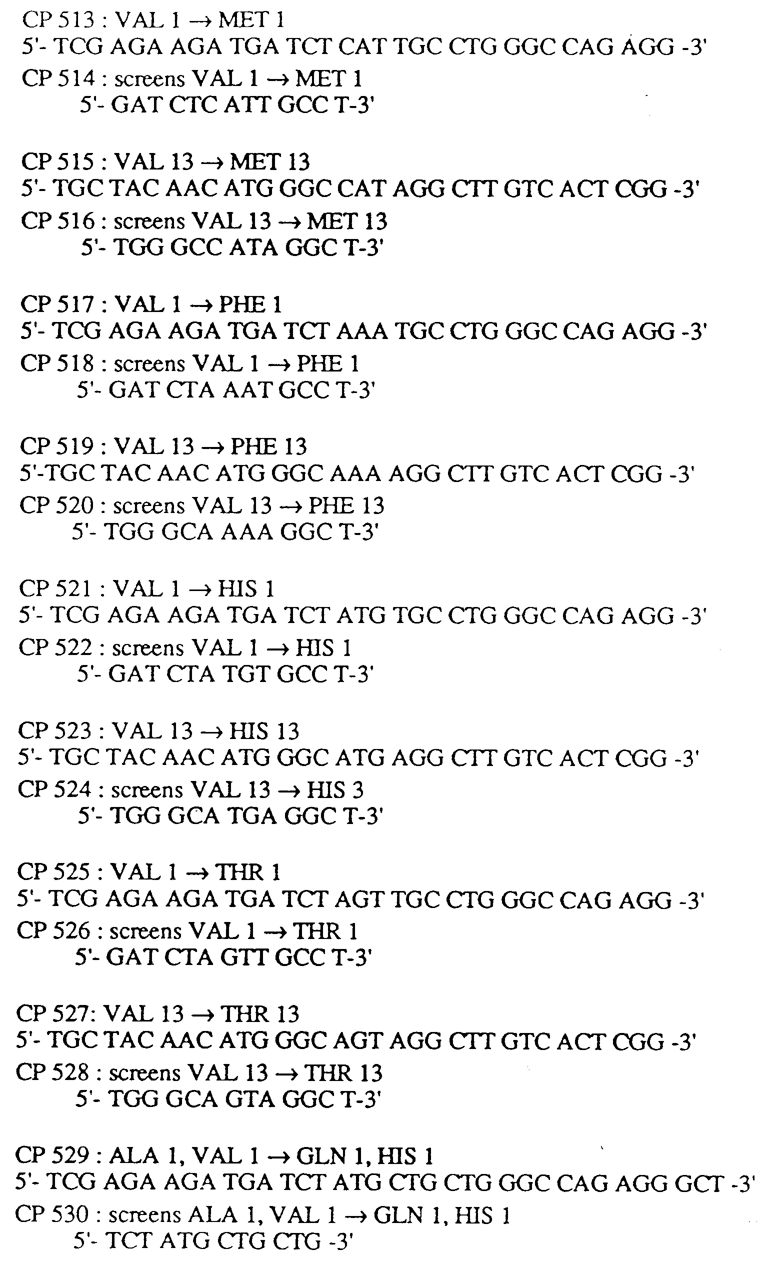

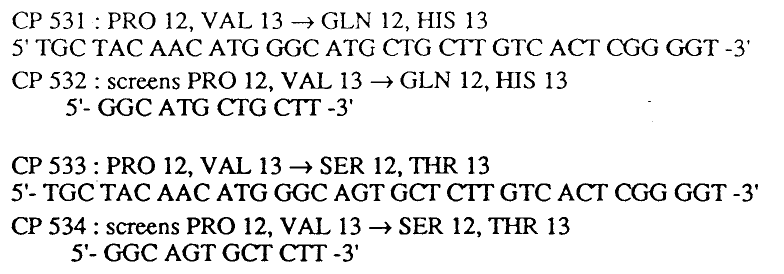

The oligonucleotides are kinased using the following reaction solution and conditions: 3 ul 10 x KB buffer, 3 λ 10 mM rATP (1:10 dilution of 0.1 M rATP stock), 2 λ mutagenic oligonucleotide (100 pmole/λ), 21 λ H₂O, and 1 λ polynucleotide kinase (10 units/λ). The reaction is run at 37°C for 45 minutes, and then at 65-68°C for 5 minutes. Next, 24 λ of the kinased oligonucleotide is diluted with 56 λ of H₂O to give 2 pmole/λ.

The gapped duplex is formed as described below, followed by annealing the oligonucleotides. The following reagents are combined in a total volume of 40 λ: 8 λ 5 x GDB buffer, 0.50 pmole ssDNA, and 0.10 pmole Hinc II linearized M 13 GAP RF DNA. 10 λ is removed for future use, and the remaining 30 λ is treated sequentially as follows: 100°C for 3 minutes, 65°C for 5 minutes, followed by cooling to room temperature for 30 minutes, and then placing the reaction mixture on ice. Next, 10 λ of gapped duplex and 10 λ of control ungapped material is subject to electrophoresis on a agarose gel to check gapped duplex formation. Assuming the gel shows the presence of a third band, the gapped duplex has formed and the kinased oligonucleotides can be annealed to the duplex by combining 16 λ of gapped duplex reaction mixture, and 4 λ of diluted kinased oligonucleotide, and heating the mixture to 65°C for 3 minutes, followed by cooling to room temperature for 20 minutes.

The heteroduplex is completed by the appropriate extension and ligation reactions consisting of combining the following reagents in a total volume of 40 λ: 10 λ gapped duplex and primer, 4 λ 10 x PEL buffer, 4 λ dNTP's (0.25 mM solution made from 10 mM stocks, 3 λ ATP (10 λ of 0.1 M ATP stock + 1490 λ H₂O = 0.662 mM), 17 λ H₂O, 1 λ Klenow (5 u/λ), and 1 λ T4 DNA ligase (0.6 Weiss u/λ, diluted stock with 1 x PEL). The reaction is conducted at 16°C for 2 hours, followed by transformation of 10 λ of the extension/ligation mixture into 200 λ of thawed competent HB2154 cells. The cells are kept at 0°C for 30 minutes, and then 42°C for 1.5 minutes, followed by plating various volumes of the transformation mix (e.g., 50 λ, 10 λ, etc.) with 100 λ of fresh overnight culture of HB2151 cells + 3.0 λ of soft agar.

The resulting plaques are screened using the plaque hybridization procedure. While a variety of such procedures are known, a description of the preferred procedure follows. Plates are replicated onto duplicate nitrocellulose filter papers (S & S type BA-85) and the DNA fixed to the filter by sequential treatment for 5 minutes with 0.5 N NaOH plus 1.5 M NaCl; 1.0 M NaCl plus 0.5 M Tris-HCl pH 7.4; and 2 x SSC (standard saline citrate). Filters are air dried and baked at 80°C for 2 hours, in vacuo.

The duplicate filters are prehybridized at 55°C for 2 hours with 10 ml per filter of DNA hybridization buffer, 5 x SSC, pH 7.0, 5 x Denhardt's solution (polyvinylpyrrolidone, plus Ficoll and bovine serum albumin; 1 x 0.02% of each), 50 mM sodium phosphate buffer at pH 7.0, 5 mM EDTA, 0.1% SDS, and 100 µg/ml yeast RNA. The prehybridization buffer is removed and the samples hybridized with the appropriate kinased probe, that is to say kinased oligonucleotides as shown above, under conditions which depend on the stringency desired. About 2 x 10⁶ cpm/ml total is used. Typical moderately stringent conditions employ a temperature of 42°C plus 50% formamide for 24-36 hours with 1-5 ml/filter of DNA hybridization buffer containing probe. For higher stringencies high temperatures and shorter times are employed. The preferred hybridization conditions consists of hybridizing the probes to the filters in 5 x SSC, Denhardt's solution, 50 mM NaPO₄, pH 7.0, 5 mM EDTA, 0.1% SDS, and 100 mg/ml yeast RNA at 10° below the T_{M} of the oligonucleotide used to do the screening. Next, the filters are washed twice, 30 minutes each wash, at room temperature with 2 x SSC, 0.1% SDS , then washed once with 2 x SSC and 0.1% SDS at 5°C below the T_{M} of the oligonucleotide used to screen, and air dried. Finally, the filters are autoradiographed at -70°C for 36 hours. Autoradiography reveals those plaques containing the virus that carries the muteins of interest.

In addition to constructing muteins wherein valine at position 2 and/or 13 have been deleted or substituted, large deletion muteins may be produced that encompass the two predominate cleavage sites of 26 kd TNF. A preferred embodiment mutein lacks the amino acids spanning the region -9 to +14, as shown in Figure 1. This mutein was constructed using the materials and methods described above and the oligonucleotide, CP375 which has the following sequence:
5'-GTTTGCTACAACATGGAGGTCCCTGGGGGA-3'

### C. Protein/Peptide Inhibitors

Peptides having the following amino acid sequences are synthesized by the solid-phase method, described in detail by Merrifield, R.B (1985) in Science, 232:341-347: Gln-Ala-Val-Arg-Ser-Ser-Ser; Gln-Ala-Val-Arg-Ser-Ser-Ser-Arg-Thr-Pro-Ser-Asp-Lys-Pro-Val-Ala; Pro-Leu-Ala-Gln-Ala-Val-Arg-Ser-Ser-Ser-Arg-Thr-Pro-Ser-Asp-Lys-Pro-Val-Ala-His-Val-Val-Ala; Arg-Thr-Pro-Ser-Asp-Lys-Pro-Val-Ala-His-Val-Val-Ala; and Pro-Leu-Ala-Gln-Ala-Val-Arg-Ser-Ser-Ser-Arg-Thr-Pro. A Biosearch 9500 automated peptide machine is used with hydrogen fluoride cleavage, and purification by preparative HPLC using a Waters Delta Prep 3000 instrument, on a 15-20 µm Vydac C4 PrepPAK column.

That these peptides inhibit convertase activity is shown by performing the assay described above in the presence of varying amounts of each peptide. Gel electrophoresis and western blotting of the reaction mixture shows an inhibition of conversion of the 26 kd TNF to the 17 kd form.

### Example 4

### Protective Effect of Convertase Inhibitors in the Treatment of Sepsis

Compounds that are effective inhibitors of convertase activity are shown to prevent sepsis in a baboon model system as follows. Anti-convertase antibody, murine, human, or recombinant, at a concentration of 5 mg/kg is administered in a single I.V. bolus 60 minutes before the animals are challenged with a lethal dose of E. coli, and 2 mg/kg simultaneously with the E. coli challenge. The antibody is administered in a physiologically balanced salt solution, and about 4 x 10¹⁰ E. coli organisms are used. The E. coli dose is infused over a two hour period. Animals that receive the antibody are protected for at least 7 days, whereas control animals that are administered only the balanced salt solution expire within 16 to 32 hours.

Similar protection is attributable to the TNF mutein convertase inhibitors shown in Example 3. The muteins are administered at a concentration of 5 mg/kg in a single I.V. bolus 60 minutes before the animals are challenged with 4 x 10¹⁰ E. coli organisms. The baboons also receive 2 mg/kg of the muteins simultaneously with the E. coli challenge.

Finally, the peptides shown in Example 3, that is, Gln-Ala-Val-Arg-Ser-Ser-Ser and, Gln-Ala-Val-Arg-Ser-Ser-Ser-Arg-Thr-Pro-Ser-Asp-Lys-Pro-Val-Ala are tested as described above and yield similar protective effects.

The present invention has been described, inter alia, with reference to specific work. However, this application is intended to cover those changes and substitutions which may be made by those skilled in the art without departing from the spirit and the scope of the appended claims.

## Claims

1. Substantially purified TNF convertase, which is inhibited by one or more compounds selected from the group consisting of 3,4-dichloro-isocoumarin, elastinal and (1-((3-((acetyloxyl)-7-methoxy-8-oxy-8-oxo-5-thio-1-azabicyclo[4.2.0]oct-2-en-2-yl)carbonyl)morphine,S,S-dioxide,(6R-cis).

2. Substantially purified TNF convertase as described in claim 1, wherein said convertase is of membrane associated origin and converts TNF having a molecular weight of about 26,000 to one or more lower molecular weight TNF species.

3. Substantially purified TNF convertase as described in claim 2, wherein said lower molecular weight TNF species has a molecular weight of about 17,000.

4. Substantially purified TNF convertase as described in claims 1, 2 or 3 derived from HL 60 cells.

5. A method of identifying prophylactics or therapeutics of an autoimmune disease, comprising the steps of:
(a) contacting TNF having a molecular weight of about 26,000 with an effective amount of TNF convertase, optionally said TNF and TNF convertase being in solution with a pH of about 7.0 and/or said TNF convertase being derived from HL60 cells;
(b) measuring the conversion of TNF having a molecular weight of about 26,000 to one or more lower molecular weight TNF species, optionally wherein said one or more lower molecular weight TNF species have molecular weights selected from about 17,000 and 15,000;
(c) repeating steps "a" and "b" above, and further including a molecule sought to be identified as a prophylactic or therapeutic of an autoimmune disease; and
(d) measuring the inhibition of said conversion of said TNF having a molecular weight of about 26,000 to one or more lower molecular weight TNF species.

6. The method of claim 5, wherein measuring the inhibition of said convertase comprises the steps of:
(a) labeling said TNF with a molecular weight of about 26,000 with a suitable label, optionally wherein said label is selected from radionuclides, fluors, or enzymes.

7. The method of claims 5 or 6 wherein said autoimmune disease is arthritis.
